(19) Europäisches Patentamt
European Patent Office
Office européen des brevets

(11) **EP 2 127 596 A1**

(12) **EUROPEAN PATENT APPLICATION**

(43) Date of publication:
**02.12.2009 Bulletin 2009/49**

(51) Int Cl.:
*A61B 5/00* (2006.01)     *A61B 5/07* (2006.01)
*A61B 5/053* (2006.01)

(21) Application number: **08157210.9**

(22) Date of filing: **29.05.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Nederlandse Organisatie voor Toegepast-Natuurwetenschappelijk Onderzoek TNO 2628 VK Delft (NL)**

(72) Inventors:
• **Erinc, Müge**
  **5641GB Eindhoven (NL)**
• **Sillekens, Wilhelmina Hubertina**
  **6049HA Herten (NL)**
• **Kampermann, Nicodemus Frederikus**
  **5737HC Lieshout (NL)**

(74) Representative: **Hatzmann, Martin et al Vereenigde Johan de Wittlaan 7 2517 JR Den Haag (NL)**

(54) **Implantable sensor, sensor assembly comprising such a sensor, implant comprising such an implantable sensor or sensor assembly and method of monitoring a condition of an in-vivo implant or of tissue surrounding said implant**

(57)     The invention relates to an implantable sensor adapted for monitoring a condition of an in-vivo implant or of tissue surrounding said implant, wherein the sensor comprises means for monitoring said condition, the means comprising an electrical circuit with at least one resistance element, the electrical circuit in use being in contact with implant material and adapted to carry a current generated by a voltage difference applied to the electrical circuit, wherein the condition can be determined by measuring said current and calculating the equivalent resistance of the electrical circuit based on said measured current. The invention further relates to a sensor assembly comprising such a sensor, to an implant comprising such an implantable sensor or sensor assembly and a method for monitoring a condition of an in-vivo implant or of tissue surrounding the implant.

Fig. 1

EP 2 127 596 A1

## Description

The invention relates to an implantable sensor adapted for monitoring a condition of an in-vivo implant or of tissue surrounding said implant.

It is often practically difficult to monitor the condition of an in-vivo implant, such as deformation of the implant, degradation time of the implant, or the condition of the surrounding tissue related to the presence of the implant in said tissue, i.e. inflammation. Multiple surgical operations can be necessary to discover and monitor said conditions. In order to avoid these operations, implantable sensors have been developed. Implantable sensors provide information about the implant, such as the amount of movement of the implant relative to the bone, the biological fixation rate of the implant and bone tissue, etc. Known drawbacks of said sensors are the necessity to replace sensing chemicals, mechanical vulnerability, the necessity to remove the sensor, etc.

Therefore, the present invention aims to provide an improved sensor of the above-described type, wherein the disadvantages of the known sensors are partly overcome. More in particular, it is an object of the invention to provide an implantable sensor which does not need refilling and can maintain mechanical integrity for a given amount of time and that can monitor the condition of the in-vivo implant in an accurate and robust way.

To that end a sensor according to the invention is **characterized in that** the sensor comprises means for monitoring said condition, the means comprising an electrical circuit with at least one resistance element, the electrical circuit in use being in contact with implant material and adapted to carry a current generated by a voltage difference applied to the electrical circuit, wherein the condition can be determined by measuring said current and calculating the equivalent resistance of the electrical circuit based on said measured current.

Since part of the electrical circuit is in contact with the implant material and with the tissue surrounding the implant, which constitutes the corrosive medium, a sensor that is able to accurately monitor conditions of the implant and of the tissue surrounding the implant is provided. This construction of the sensor enables monitoring of the condition parameters by means of measuring changes in electrical resistance of the electrical circuit. The sensor can monitor different conditions and changes from very early to late stages of occurrence, and provide relevant data to a medical skilled person on a periodic check after a short while of occurrence of the change in said conditions. Furthermore, the dimensional tolerances are not very critical for the accuracy of measurement of the parameters for determining the condition. Consequently, the sensor provides a reliable, easy and at the same time clean way of monitoring different conditions of the implant and tissue surrounding the implant. Furthermore, in the case that a sensor permanently has to stay in a patient's body, a sensor according to the invention can accurately indicate if an implant is worn out and needs to be replaced. This decreases the health risks of frequent exposure to X-rays in order to monitor the condition of the implant.

In further elaboration of the invention, the sensor is a biodegradable sensor. Such a sensor can be implanted at the same time as the implant is implanted in a patient's body. The sensor can degrade after a certain period of time, when the sensor is not needed anymore, or when the sensor is ceased to be effective. For instance, if a biodegradable implant is used, that implant will disappear after a while depending on the kind of implant and a patient's medical situation. The biodegradable sensor will also degrade to the extent that it is completely eliminated from the body, preferably leaving no residue in the body. Consequently, no second or further surgery will be needed to remove an ineffective sensor from a patient's body. This is advantageous since every surgery may involve risks for a patient's health. Furthermore, total medical costs will decrease, which is advantageous for health care costs.

Preferably, the components of the sensor and/or the implant material comprises a biodegradable metal or metal alloy, such as magnesium, iron, magnesium alloys and/or iron alloys with Al, Ca, F, Fe, Li, Mg, Mn, rare earth elements, Zn, Zr, or the like or any combination thereof. The mentioned materials will be resorbed in the body completely after a certain period of time. In another embodiment of the invention, the sensor can be used with an implant of a non-degradable material such as a Ti alloy, stainless steel or Co-Cr alloy. The components can at least comprise a circuit wire and the at least one resistance wire.

The at least one resistance element in the electrical circuit of the sensor preferably has a high electrical resistance thereof the electrical conductivity is significantly temperature dependent.

In order to further enable accurate monitoring, the electrical circuit according to a further elaboration of the invention has a higher corrosion resistance than the implant material. Consequently, the sensor continues operating in an accurate way during degradation of the implant. After the implant has degraded, the sensor also degrades in order to leave no residue in the patient's body.

According to another aspect of the invention, the sensor comprises an insulating body that substantially encapsulates the electrical circuit, wherein the ends of the electrical circuit extend from the insulating body, wherein the insulating body has a higher corrosion resistance than the implant material. If the sensor is completely biodegradable, the insulating body preferably is of a biodegradable polymer. The sensor comprising said body has a further advantage in that the rigid plate structure provides mechanical stability.

It is possible, according to a further elaboration of the invention, that the insulating body comprises a receiving section for receiving a monitoring element of the implant material. According to a further elaboration of the invention, a sensor assembly comprising an above described sensor and a monitoring element of the implant material is provided. In such an assembly, the monitoring element is a part of the electrical circuit and it extends from the sensor body such that a first end of the monitoring element in use contacts the tissue surrounding the implant. It is known that when a foreign object is implanted in a living body, depending on the foreign material and local pH, tissue might build up on the object. This tissue may grow between the implant and the sensor, thereby disturbing the measurement of the condition parameters of said implant. By placing the monitoring element into the sensor, more particularly in the receiving section of the sensor, and making it a part of the circuit, the sensor can directly monitor the condition parameters without influence of the tissue surrounding the sensor and the implant. Consequently, accurate measurement of the condition parameters is further enhanced.

In further elaboration of the invention, the electrical circuit comprises a plurality of resistance elements that are spaced apart and which are connected in parallel to the electrical circuit, at least to a circuit wire of the electrical circuit, with a first end and to the implant material with a second end, wherein the electrical circuit and the resistance elements preferably have a substantially similar corrosion resistance. Such a construction of the sensor provides a clear determination of a change in resistance at the exact moment that a resistance element disconnects from the monitoring element.

According to an alternative embodiment of the invention, the electrical circuit comprises a plurality of resistance elements in series, wherein the resistance elements are encapsulated in an insulating material, wherein the resistance elements, the insulating material and the implant preferably have a substantially similar corrosion resistance which is lower than a corrosion resistance of a circuit wire of the electrical circuit.

In another alternative embodiment of the sensor according to the invention, the electrical circuit comprises a resistance element, wherein the electrical circuit and the resistance element have a substantially higher corrosion resistance rate than the implant. Such alternative embodiments of the sensor provide a construction of the sensor integrateable in the implant, thereby reducing the amount of parts of the sensor resulting in reduction of manufacturing time and costs.

The invention further relates to an implant comprising the above-described sensor or sensor assembly, wherein the electrical nodes extend from the implant, wherein the implant contacting the electrical circuit is adapted to in use contact the tissue surrounding the implant. Preferably, the implant is made of a biodegradable material.

Finally, the invention relates to a method for monitoring a condition of an in-vivo implant or of tissue surrounding said implant, wherein the method comprises the steps of:

- implanting an implant and a sensor according to any one of claims 1-9 or a sensor assembly according to claim 10 into a body of a patient;
- applying a voltage difference to excite the electrical circuit of the sensor to generate a current;
- measuring the current;
- calculating the equivalent resistance of the electrical circuit based on said measured current
- determining the condition based on the calculated equivalent resistance.

By determining the electrical resistance of the electrical circuit of the sensor, the rate of corrosion of the implant can be determined. Moreover, by determining the thermal shift in the electrical resistance of the electrical circuit, the occurrence of inflammation in the tissue surrounding the implant can be determined.

Such an implant and a method for monitoring a condition of an in-vivo implant or the tissue surrounding the implant provide similar advantages and effects as described earlier with the implantable sensor according to the invention.

Further advantageous embodiments of a sensor according to the invention, an implant provided therewith and a method for monitoring a condition of an in-vivo implant or of tissue surrounding said implant are set forth in the dependent claims.

To explain the invention, exemplary embodiments thereof will hereinafter be described with reference to the accompanying drawings, wherein:

FIG. 1 schematically shows a first embodiment of a sensor according to the invention; and
FIG. 2 schematically shows an implant comprising a sensor according to a first embodiment of the invention.
FIG. 3 schematically shows an implant comprising the sensor according to a second embodiment of the invention; and
FIG. 4 schematically shows an implant comprising a sensor according to a third embodiment of the invention.

It is noted that similar parts in the different figures are referred to by similar reference numerals.

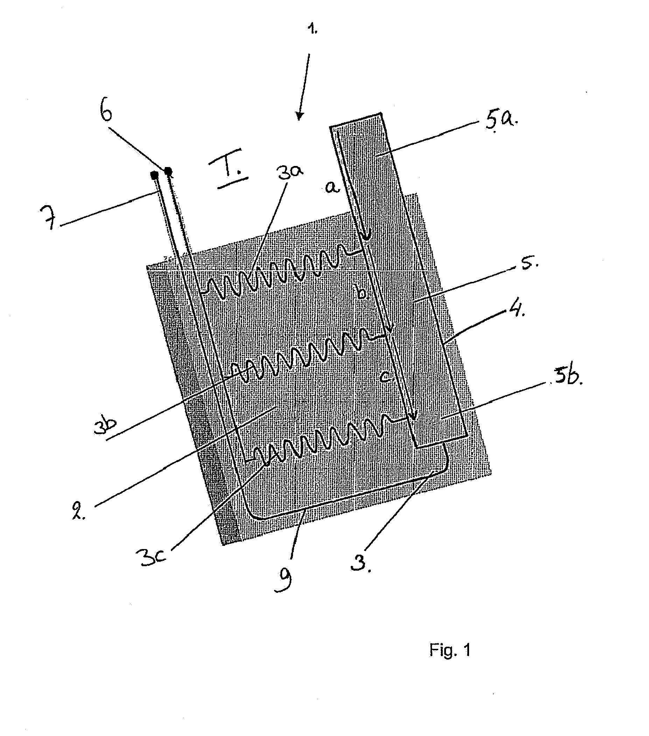

In Figure 1 an implantable sensor 1 according to the invention is shown. The sensor 1 comprises a body 2. The body 2 can be of an electrically insulating material such as a biodegradable polymer. Preferably, the biodegradable material of the body 2 is a biodegradable polymer, for instance one of poly-1-lactic acid (PLLA), polyglycolic acid (PGA), poly-D,L-lactide.glycolide copolymer (PDLA), polyaprolactine (PCL) or the like. The body 2 substantially encapsulates the electrical circuit 3. The body 2 further comprises a receiving section 4 wherein a monitoring element 5 is provided. The body 2 can be of two polymer sheets where in between the electrical circuit 3 and the monitoring element 5 are provided. The monitoring element 5 is of the same material as the material of the implant (not shown) and thus comprises a similar corrosion resistance. The circuit wire 9 is of a material having a corrosion resistance that is higher than that of the implant material. The ends of the circuit wire 9, which ends define electrical nodes (6, 7) are in contact with the tissue (T) surrounding the implant and the sensor 1. The circuit wire 9 and the monitoring element 5 both comprise a biodegradable metal or metal alloy, such as magnesium, iron, magnesium alloys and/or iron alloys with Al, Ca, F, Fe, Li, Mg, Mn, rare earth elements, Zn, Zr or the like or any combination thereof.

The circuit wire 9 comprises one or more (here three as an example) resistance elements 3a, 3b, 3c that are spaced apart and all are connected in parallel to the circuit wire 9 with a first end and connected to the monitoring element 5 with a second end. The circuit wire 9 and the resistance elements 3a, 3b, 3c preferably have a similar corrosion rate. The monitoring element 5 together with the resistance elements 3a, 3b, 3c and the circuit wire 9 form the electrical circuit 3.

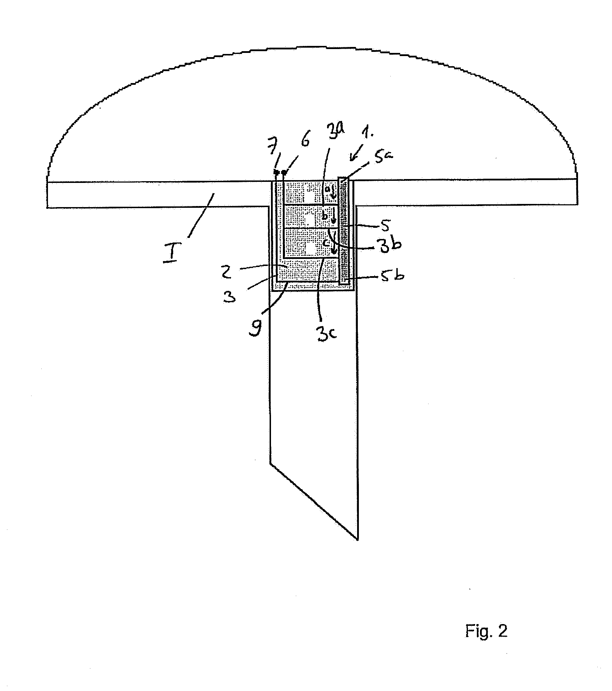

The sensor 1 can be connected to an implant before or after it is implanted in the body. The sensor 1 can also be embedded in the implant I (see Fig. 2). When the sensor 1 is connected to an implant I, the sensor 1 will not be able to drift easily in the patient's body. Furthermore, since the monitoring element 5 is provided close to the implant I, the monitoring element 5 will be exposed to substantially the same physiological environment as the implant, which further increases the accuracy of the measurements taken by the sensor 1.

For determining for instance the corrosion rate of the implant I, the corrosion rate of the monitoring element 5 is monitored. In use, thus in-vivo, the monitoring element 5 corrodes due to the contact with the physiological environment of the implant. Consequently, the first end 5a of the monitoring element 5 will degrade, thereby decreasing the total length of the monitoring element 5. In other words, material of the monitoring element 5 on the side 5a that is facing away from the body 2 will degrade first.

When a voltage difference is applied, a current will be generated in the electrical circuit 3. The magnitude of the generated current can be determined outside the patient's body for instance by using an electromagnetic field detector. It is also possible to extend the nodes 6, 7 to the skin, for instance by biocompatible and/or biodegradable wires extending from the sensor, such that wired measurements can be taken. Wired measurement can for instance be preferred for orthopaedic implants where the implant is close to skin. From the current, the resistance of the electrical circuit 3 can be calculated as explained later.

When a certain amount of material (represented by arrow a) of the monitoring element 5 has degraded, the first resistance element 3a will disconnect from monitoring element 5. Since the electrical circuit 3 forms a switch on/off type of circuit, the equivalent resistance of the electrical circuit 3 can only have certain values. Assuming that the electrical circuit 3 comprises three identical resistance elements 3a, 3b, 3c having resistance R in parallel, the initial resistance R°, at a certain reference temperature is determined by:

$$\frac{1}{R°} = \frac{1}{R} + \frac{1}{R} + \frac{1}{R}$$

If the resistance elements 3a, 3b, 3c are consecutively disconnected from the circuit 3, the instantaneous equivalent resistance R' of the circuit 3 can have the following values:

$$R' = \frac{R°}{3},$$

when three resistance elements 3a, 3b, 3c are still connected to the monitoring element 5;

$$R' = \frac{R°}{2},$$

when one resistance element 3a is disconnected (thus the monitoring element 5 has degraded over length a) and two resistance elements 3b, 3c are still connected;

$$R' = R°,$$

when two resistance elements 3a, 3b are disconnected (thus the monitoring element 5 has degraded over length a+b) and one resistance element 3c is still connected; and

$$R' = \infty,$$

when none of the resistance elements 3a, 3b, 3c is connected to the monitoring element 5 since the monitoring element 5 has degraded over length a+b+c.

Calibration of the sensor 1 needs to be done before the implantation by laboratory tests. An empirical correlation between the corrosion rate of the implant I and the corrosion rate measured by the sensor 1 can be derived experimentally.

The initial number of resistance elements 3a, 3b, 3c comprised in the electrical circuit determines the accuracy of corrosion rate measurement. However, there is an optimum number regarding for instance space limitations and the fact that a high number of resistance elements 3, 3b, 3c might conflict with the temperature monitoring as explained below.

Inflammation around an implant can be detected by monitoring the local temperature of tissue T. The local temperature can be detected by the sensor 1 according to the invention, as follows. As explained above, at a certain temperature $T_{ref}$ (normal in-vivo temperature), R' can have only certain values. Deviation from these values indicates a temperature related shift in the equivalent resistance, given by the following relationship:

$$T = T_{ref} + \frac{\dfrac{R'}{R_{ref}} + 1}{\alpha}$$

wherein a is the average temperature coefficient of resistance in a certain temperature range. To be able to measure the temperature difference, the resistance change caused by the temperature difference should be less than the resistance change caused by the disconnection of the individual resistance elements 3, 3b, 3c. This rule of thumb can be formulated as:

$$\Delta R_{temp} < \frac{R°}{n}$$

wherein n is the number of resistance elements. This range becomes smaller as the number of resistance elements increases.

The sensor 1 can be attached to an implant I after the implant I is placed in a patient's body. In this way, more working space around the implant I is created for the surgeon, without the sensor being in the way. However, the sensor 1 can already be connected to the implant I for instance during the manufacturing process, or can be embedded in the implant I as can be seen in Figure 2.

In Figure 2 an implant I, in this case a bone screw, comprising a sensor 1 as above described is schematically shown. The sensor 1 is completely embedded in the bone screw. The electrical nodes 6, 7, thus both ends of the electrical circuit 3, and also the first end 5a of the monitoring element 5 of the implant material extend from the outer surface of the bone screw in a direction away from the implant I into the tissue T surrounding the implant I. In use, the electrical nodes 6, 7 and the first end 5a of the monitoring element 5 are in contact with human tissue T surrounding the implant I. The working principles of this second embodiment of the sensor 1 according to the invention are the same as explained in Figure 1 and therefore will not be explained again.

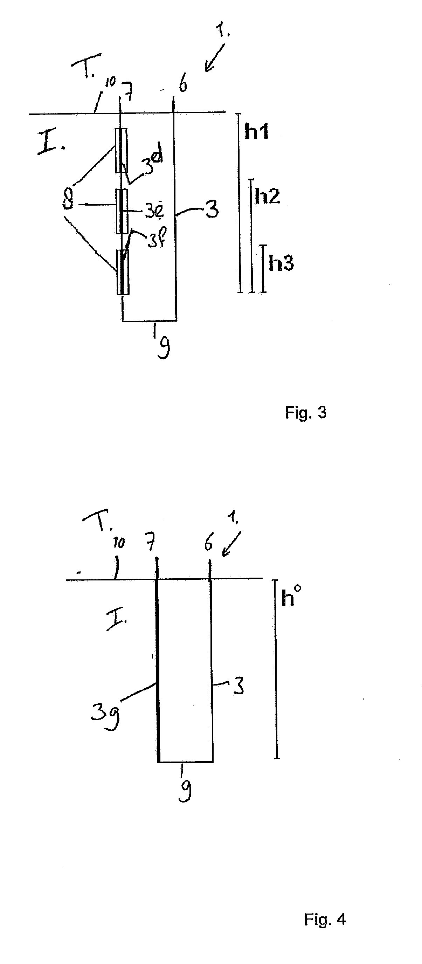

Figure 3 shows an implant I comprising a sensor 1 according to a second embodiment of the invention. The sensor 1 is integrated with the implant I. The electrical circuit 3 comprises resistance elements 3d, 3e, 3f in series with the circuit wire 9. The separate resistance elements 3d, 3e, 3f, are encapsulated by insulating material 8, at least by a material that is an electrical insulator at the voltages used to activate the sensor 1. The insulating material 8 has a similar corrosion rate as the implant material. Due to this construction of the sensor 1, the metallic implant I can not short circuit the sensor 1. The material of the sensor 1 degrades at the same rate as the material of the implant I. The insulating material 8 can be of a biodegradable polymer, hydroxyapatite (HA) or a bio-compatible powder or gel or the like. The resistance elements 3d, 3e, 3f are connected in series in the electrical circuit 3. The circuit wire 9 has a higher corrosion resistance rate than the other parts of the sensor 1. Since the resistance elements 3d, 3e, 3f will corrode at the same rate as the implant I, an extra monitoring element of the implant material will not be needed. In this embodiment of the sensor 1, the current is applied to the electrical circuit in the same way as mentioned before. The total resistance of the sensor 1 to be measured a time t=0 is:

$$R° = R_1 + R_2 + R_3$$

The remaining life of the implant I can be calculated as follows:

$$R' = \begin{cases} 3R \\ 2R \\ R \\ 0 \end{cases}$$

wherein 3R represents the implant length h1 being intact, 2R represents h2 being intact, R represents h3 being intact and 0 represents that length h1 has corroded.

In Figure 4, a further embodiment of the sensor 1 according to the invention is shown. In this embodiment the sensor 1 is again completely embedded in the implant I. A single resistance element 3g is placed in the electrical circuit 3. The resistance element 3g and the circuit wire 9 of the electrical circuit 3 have a significantly higher corrosion resistance than the implant material. In this embodiment, neither a monitoring element nor an insulating material is necessary, which is advantageous regarding ease in manufacturing. The sensor 1 works as follows. When the implant I is intact, the measured resistance is minimum, since the metallic implant I short-circuits the resistance element 3g. After being exposed to a corrosive medium, i.e. the tissue T surrounding the implant I, the implant I will corrode in time. As corrosion advances, non-conductive corrosion product will surround the electrical circuit 3, thereby increasing the measured resistance when a current is applied to the electrical circuit 3. The current is applied in the same way as mentioned before. The long resistance 3g perpendicular to the implant surface 10 will ensure that the implant I is acting as a rheostat. Such a sensor 1 provides a more accurate measurement of the advancement of corrosion. If the original resistance is R° spanning a length of h°, and the measured resistance is R, the corroded implant length is represented by:

$$h_{corr} = h^{\circ} * \frac{R}{R^{\circ}}$$

The sensor 1 integrated in the implant I according to the second and third embodiment of the invention (see Fig. 3 and Fig. 4), has a further advantage such that due to the direct sensing from the implant, calibration of the sensor is not necessary. Because of the relatively large implant surface 10, the local pH around the implant is almost instantly at equilibrium with its surroundings. However, the corrosion products at the monitoring element are confined to a narrow space and the chemical equilibrium is dependent on the diffusion rate.

The invention is not in any way limited to the exemplary embodiments presented in the description and drawings. All combinations (of parts) of the embodiments shown and described are explicitly understood to fall within the scope of the invention. Moreover, many variations are possible within the scope of the invention, as outlined by the claims.

## Claims

1. An implantable sensor adapted for monitoring a condition of an in-vivo implant (I) or of tissue (T) surrounding said implant (I), wherein the sensor (1) comprises means for monitoring said condition, the means comprising an electrical circuit (3) with at least one resistance element (3a-3g), the electrical circuit (3) in use being in contact with implant material and adapted to carry a current generated by a voltage difference applied to the electrical circuit, wherein the condition can be determined by measuring said current and calculating an equivalent resistance of the electrical circuit based on said measured current.

2. An implantable sensor according to claim 1, wherein at least one end of the electrical circuit (3), which at least one end defines an electrical node (6, 7), is adapted to be in use in contact with the tissue (T) surrounding the implant (I).

3. An implantable sensor according to any one of the preceding claims wherein the sensor (1) is a biodegradable sensor.

4. An implantable sensor according to any one of the preceding claims, wherein the electrical circuit (3) has a higher corrosion resistance than the implant material.

5. An implantable sensor according to any one of the preceding claims, wherein the sensor (1) comprises an insulating body (2) that substantially encapsulates the electrical circuit (3), wherein the ends of the electrical circuit (3) extend from the insulating body (2), wherein the insulating body (2) has a higher corrosion resistance than the implant material.

6. An implantable sensor according to claim 5, wherein the insulating body (2) comprises a receiving section (4) for receiving a monitoring element (5) of the implant material.

7. An implantable sensor according to claim 5 or 6, wherein the electrical circuit (3) comprises a plurality of resistance elements (3a, 3b, 3c) that are spaced apart and which are connected in parallel to the electrical circuit (3), at least to a circuit wire(9) of the electrical circuit (3), with a first end and to the implant material with a second end, wherein the electrical circuit (3) and the resistance elements (3a, 3b, 3c) preferably have a substantially similar corrosion resistance.

8. An implantable sensor according to any one of claims 1-4, wherein the electrical circuit (3) comprises a plurality of resistance elements (3d, 3e, 3f) in series, wherein the resistance elements (3d, 3e, 3f) are encapsulated in an insulating material (8), wherein the resistance elements (3d, 3e, 3f), the insulating material (8) and the implant (I) preferably have a substantially similar corrosion resistance which is lower than a corrosion resistance of a circuit wire (9) of the electrical circuit (3).

9. An implantable sensor according to any one of claims 1-4, wherein the electrical circuit (3) comprises a resistance element (3g), wherein the electrical circuit (3) and the resistance element (3g) have a substantially higher corrosion resistance rate than the implant.

10. A sensor assembly comprising a sensor according to any one of claims 5-7 and a monitoring element (5) of the implant material, wherein the monitoring element (5) is connected to the electrical circuit (3) and wherein a first end (5a) of the monitoring element (5) extends from the insulating body (2) and is adapted for contacting the tissue (T) surrounding the implant (I).

11. An implant comprising a sensor according to any one of claims 1-9 or a sensor assembly according to claim 10, wherein the electrical nodes (6, 7) extend from the implant (I), wherein the implant (I) contacting the electrical circuit (3) is adapted to in use contact the tissue (T) surrounding said implant (I).

12. An implant according to claim 11, wherein the implant (I) is made of a biodegradable material.

13. A method for monitoring a condition of an in-vivo implant or of tissue surrounding said implant, wherein the method comprises the steps of:

- implanting an implant (I) and a sensor (3) according to any one of claims 1-9 or a sensor assembly according to claim 10 into a body of a patient;
- applying a voltage difference to excite the electrical circuit of the sensor (1) to generate a current;
- measuring the current;
- calculating the equivalent resistance of the electrical circuit based on said measured current
- determining the condition based on the calculated equivalent resistance

14. A method according to claim 13, wherein the condition is a rate of corrosion of the implant (I) and/or occurrence of inflammation of the tissue (T) surrounding the implant (I).

15. A method according to claim 13 or 14, wherein the current is measured wireless from outside the patient's body, for instance by an electromagnetic field detector.

16. A method according to claim 13 or 14, wherein the current is measured wired from a patient's skin, for instance by biocompatible and/or biodegradable wires extending from the sensor (1) to the skin.

Fig. 1

Fig. 2

Fig. 3

Fig. 4

Europäisches
Patentamt

European
Patent Office

Office européen
des brevets

## PARTIAL EUROPEAN SEARCH REPORT

**Application Number**

which under Rule 63 of the European Patent Convention EP 08 15 7210
shall be considered, for the purposes of subsequent
proceedings, as the European search report

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/090159 A (MEDTRONIC INC [US]; CHEN KAIMIN [US]; SCHMIDT CRAIG L [US]; MERRITT DO) 9 August 2007 (2007-08-09) | 1,2,6, 10,11 | INV. A61B5/00 A61B5/07 |
| Y | * abstract * <br> * page 2, line 12 - line 16 * <br> * page 27, line 23 - page 29, line 11; figures 12A,12B,13A,13B * <br> ----- | 4,5,7-9 | ADD. A61B5/053 |
| X | US 2003/199783 A1 (BLOOM MATTHEW [US] ET AL) 23 October 2003 (2003-10-23) <br> * abstract * <br> * paragraph [0025] - paragraph [0029] * <br> * paragraph [0041] * <br> * paragraph [0070] - paragraph [0071] * <br> * figure 2G * <br> ----- | 1 | |
| Y | EP 1 177 811 A (TERUMO CORP [JP]) 6 February 2002 (2002-02-06) <br> * abstract * <br> * paragraph [0049] - paragraph [0054] * <br> ----- | 4,5,7-9 | |

-/--

**TECHNICAL FIELDS SEARCHED (IPC)**

A61B

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 23 October 2008 | Tommaseo, Giovanni |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

**EP 2 127 596 A1**

Europäisches Patentamt
European Patent Office
Office européen des brevets

## PARTIAL EUROPEAN SEARCH REPORT

Application Number

EP 08 15 7210

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| A | EP 0 344 770 A (ISRAEL STATE [IL]; YEDA RES & DEV [IL]) 6 December 1989 (1989-12-06) * abstract * * column 5, line 41 - column 7, line 52 * * claims 18-20 * ----- | 1-12 | |
| A | US 6 901 294 B1 (WHITEHURST TODD K [US] ET AL) 31 May 2005 (2005-05-31) * abstract * * figures 3,4 * ----- | 1 | |
| | | | TECHNICAL FIELDS SEARCHED (IPC) |

EPO FORM 1503 03.82 (P04C10)

12

Europäisches
Patentamt

European
Patent Office

Office européen
des brevets

**INCOMPLETE SEARCH**
**SHEET C**

Claim(s) searched completely:
        1-12

Claim(s) not searched:
        13-16

Reason for the limitation of the search (non-patentable invention(s)):

Article 53 (c) EPC - Method for treatment of the human or animal body by surgery

## ANNEX TO THE EUROPEAN SEARCH REPORT
## ON EUROPEAN PATENT APPLICATION NO.

EP 08 15 7210

This annex lists the patent family members relating to the patent documents cited in the above-mentioned European search report.
The members are as contained in the European Patent Office EDP file on
The European Patent Office is in no way liable for these particulars which are merely given for the purpose of information.

23-10-2008

| Patent document cited in search report | | Publication date | Patent family member(s) | | Publication date |
|---|---|---|---|---|---|
| WO 2007090159 | A | 09-08-2007 | US 2007176151 A1<br>WO 2007089980 A2 | | 02-08-2007<br>09-08-2007 |
| US 2003199783 | A1 | 23-10-2003 | US 2006047218 A1 | | 02-03-2006 |
| EP 1177811 | A | 06-02-2002 | WO 0166181 A1<br>JP 3977569 B2<br>JP 2001245991 A<br>US 2003088302 A1 | | 13-09-2001<br>19-09-2007<br>11-09-2001<br>08-05-2003 |
| EP 0344770 | A | 06-12-1989 | NONE | | |
| US 6901294 | B1 | 31-05-2005 | NONE | | |

EPO FORM P0459

For more details about this annex : see Official Journal of the European Patent Office, No. 12/82